# EUROPEAN PATENT SPECIFICATION

(11) **EP 4 169 551 B1**
(45) Date of publication and mention of the grant of the patent: **25.03.2026**
(21) Application number: 21829750.5
(22) Date of filing: 22.06.2021
(51) Int. Cl.: A61M 5/158, A61M 25/06

(54) **OUTER NEEDLE ASSEMBLY**
AUSSENNADELANORDNUNG
ENSEMBLE AIGUILLE EXTERNE

(30) Priority: 22.06.2020 JP 2020106775
(43) Date of publication of application: 26.04.2023
(73) Proprietor: Nipro Corporation, Osaka-shi, Osaka 531-8510 (JP)
(72) Inventor: SADAHIRO, Kaname, Osaka-shi, Osaka 531-8510 (JP); NAKAGAMI, Hiroyuki, Osaka-shi, Osaka 531-8510 (JP); KUDO, Tatsuya, Osaka-shi, Osaka 531-8510 (JP); KATAOKA, Naoya, Osaka-shi, Osaka 531-8510 (JP)
(74) Representative: Slingsby Partners LLP
(86) International application number: PCT/JP2021/023686
(87) International publication number: WO 2021/261500

(56) References cited:
- EP-A1- 3 662 959
- WO-A1-2019/146791
- US-A1- 2011 160 662

## Description

### TECHNICAL FIELD

The present invention relates generally to an outer needle assembly including an outer needle and a passage forming member connected to the proximal end side of the outer needle. More particularly, the present invention pertains to an outer needle assembly including a ventilation path capable of discharging air from the bore of the passage forming member to the outside, and a valve body by which the bore of the passage forming member becomes in communication or blocked.

### BACKGROUND ART

Conventionally, an outer needle assembly used for dialysis, infusion, etc. has been known. For example, like an indwelling needle disclosed in Japanese Unexamined Patent Publication No. JP-A-2015-080707 (Patent Document 1), the outer needle assembly includes a hollow outer needle, and a passage forming member such as an outer needle base connected to the proximal end side of the outer needle in a communicating state. After the outer needle being stuck in a blood vessel of a patient or the like together with an inner needle, the inner needle is removed, whereby the outer needle assembly is indwelled in the blood vessel or the like. Then, dialysis or infusion is performed by connecting an external passage such as a dialysis circuit and an infusion line to an outer needle assembly indwelled in the blood vessel or the like.

Besides, in the indwelling needle of Patent Document 1, a rubber stopper part serving as a valve body is accommodated in the outer needle base, and by opening and closing a slit valve provided in the rubber stopper part, communication and blocking of the bore of the outer needle base can be switched. Specifically, in a state where the inner needle is removed and the indwelling needle is indwelled in the blood vessel, the slit valve of the rubber stopper part closes and the bore of the outer needle base is blocked, so that blood leakage is limited. On the other hand, by connecting the external passage to the outer needle base, the slit valve of the rubber stopper opens so that the external passage communicates with the blood vessel via the bore of the indwelling needle.

### BACKGROUND ART DOCUMENT

### PATENT DOCUMENT

Patent Document 1: JP-A-2015-080707
EP 3662959 describes an indwelling needle provided with hemostatic valve, and an indwelling needle assembly. A hemostasis valve-equipped indwelling needle includes a cannula to be inserted percutaneously into a blood vessel on a distal end side thereof, a link connector on a proximal end side thereof, an internal flow path extending from the cannula to the link connector, and a hemostatic valve disposed inside the link connector. An air vent passage that allows the internal flow path to communicate with an external space is formed in the link connector further on the cannula side than the hemostatic valve, and a filter that allows gas to pass through but does not allow liquid to pass through is mounted in a compressed state on the air vent passage.
US 2011/0160662 describes systems and methods for providing a flushable catheter assembly having features to enable selective activation of fluid flow through the catheter assembly. A septum is placed within the catheter adapter of the catheter assembly and includes a pathway that is closed prior to being biased open via a septum activator also positioned within the catheter adapter. A plurality of air vent channels is interposed between the septum and the inner surface of the catheter adapter to permit "flashback" of blood during insertion of the catheter into a patient. The septum activator is advanced through the pathway of the septum as a coupler is attached to a proximal opening of the catheter adapter.

### SUMMARY OF THE INVENTION

### PROBLEM THE INVENTION ATTEMPTS TO SOLVE

Meanwhile, in the outer needle assembly indwelled in the blood vessel, before connection of the external passage, it is necessary to fill the bore with a liquid such as blood and discharge air, so as to prevent the air from entering the blood vessel. Therefore, in Patent Document 1, when the blood flows into the bore of the indwelling needle (the outer needle assembly), the air will be discharged to the outside through a gap serving as a ventilation path formed between the outer needle base and the rubber stopper part.

However, research conducted by the inventors through tests has newly revealed that when the outer needle assembly is tilted so that the needle tip points diagonally upward and puncture is performed, in some instances, air is not sufficiently discharged and remains in the bore of the outer needle assembly.

It is therefore one object of the present invention to provide an outer needle assembly of novel structure which is able to prevent air from remaining.

### MEANS FOR SOLVING THE PROBLEM

In order to prevent air from remaining in the bore of the outer needle assembly, the inventors first tried to change the shape, size, number, arrangement, etc. of the ventilation path. However, it was not possible to obtain a sufficient effect.

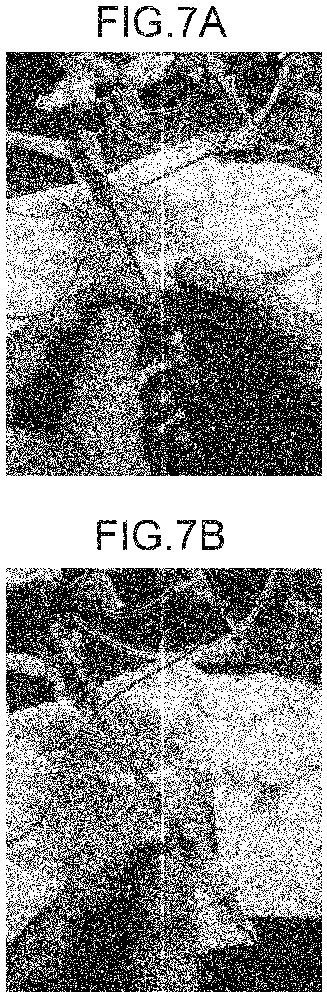

Next, the inventors observed in detail the flow mode of the liquid (corresponding to blood) in the outer needle assembly by tests. As a result, it was demonstrated that when the inner needle is pulled out from the outer needle from the state of puncture of the inner needle shown in FIG. 7A to the state shown in FIG. 7B, the liquid flowing from the needle tip of the outer needle was positioned so as to be separated to the distal end side and the proximal end side in the outer needle assembly, and discharge of air to the proximal end side was obstructed by the liquid, so that the air remained between the liquid on the distal end side and the liquid on the proximal end side.

The inventors examined the reason why the liquid introduced into the bore of the outer needle assembly was separated to the distal end side and the proximal end side with respect to the remaining air so that the air remained in the middle of the liquid. During the examination, the inventors focused on the existence of a stepped diameter-enlarged portion on the inner circumferential surface of the outer needle assembly. It was presumed by the inventors that when such a stepped diameter-enlarged portion was provided in the bore of the outer needle assembly, the cross-sectional area of the bore of the outer needle assembly through which the liquid flowed suddenly expanded on the way, whereby a region which was not filled with the liquid would be generated at the diameter-enlarged portion. Then, it was considered that the liquid reached the proximal end of the outer needle assembly while the air in the region which was not filled with the liquid was remaining, and the ventilation path was blocked by the liquid so as to obstruct discharge of the air through the ventilation path and the air remained in the middle portion of the outer needle assembly, whereby the liquid existed on the opposite sides of the remaining air. Besides, according to the review by the inventors, it was presumed that when the liquid flowed through the stepped diameter-enlarged portion in the bore of the outer needle assembly, turbulence was likely to occur due to a sudden change in the passage cross-sectional area, whereby air entrainment in the liquid occurred and the discharge of the air became insufficient, which was also one of the causes of the remaining air. Then, the inventors have completed the present invention based on such findings.
According to an aspect of the present invention, there is provided an outer needle assembly as claimed in claim 1. Additional features are provided in the dependent claims.

The invention is defined by the appended claims. The methods described herein are merely presented for illustrative purposes. Hereinafter, preferred examples for grasping the present invention will be described. However, each preferred example described below is exemplary and can be appropriately combined with each other. Besides, a plurality of elements described in each preferred example can be recognized and adopted as independently as possible, or can also be appropriately combined with any element described in other preferred examples. By so doing, in the present invention, various other preferred examples can be realized without being limited to those described below.

An example provides an outer needle assembly comprising: an inner needle; an outer needle of hollow shape through which the inner needle is inserted; a passage forming member connected to the outer needle; and a valve body arranged inside the passage forming member, a first internal space on a distal end side with respect to the valve body being longer than a second internal space on a proximal end side with respect to the valve body, wherein a ventilation path for discharging air in the first internal space to an outside communicates with the first internal space in a vicinity of the valve body, and the first internal space comprises: a puncture part formed in a portion of the outer needle, the portion puncturing a blood vessel; a tapered part formed on the proximal end side with respect to the puncture part, the tapered part having an inner diameter dimension which increases toward the proximal end side; and a small-diameter part formed on the proximal end side with respect to the tapered part, the small-diameter part having an inner diameter dimension which is smaller than or equal to that of a proximal end of the tapered part.

According to the outer needle assembly,
the cross-sectional area of the internal space at the
small-diameter part is equal to or smaller than the cross-sectional area of the internal space at the proximal end of the tapered part. Thus, when the blood flows into the small-diameter part via the puncture part and the tapered part, the blood is less likely to wrap around the air to cause partial precedence, and the blood readily flows through the entire cross section of the small-diameter part. As a result, it is unlikely that the partially preceding blood passes the small-diameter part while leaving the air, and the blood blocks the ventilation path before the air is discharged. Accordingly, the air in the small-diameter part will be pushed out to the ventilation path by the blood without remaining and discharged to the outside. Therefore, even if the needle tip is pointed diagonally upward and puncture is performed, the air is less likely to remain in the first internal space on the distal end side with respect to the valve body.

The phenomenon of remaining air is likely to occur when the ventilation path and the outer needle are largely separated from each other. Thus, the configuration capable of preventing the air from
remaining is preferably applied to an outer needle assembly in which a ventilation path and an outer needle are arranged at a large distance from each other by including a tubular member between a member holding a valve body and a member holding the outer needle or the like. Further, the configuration
is particularly preferably applied to an outer needle assembly in which
the length of the small-diameter part is longer than the distance from the distal end of the tapered part to the distal end of the small-diameter part. Additionally, a large-diameter part having an inner diameter larger than the small-diameter part is formed on the proximal end side with respect to the small-diameter part, and the large-diameter part may be adjacent to the valve body.

A second example provides an outer needle assembly comprising: an inner needle; an outer needle of hollow shape through which the inner needle is inserted; a passage forming member connected to the outer needle; a valve body arranged inside the passage forming member; and a ventilation path provided to the passage forming member and placing an internal space on a distal end side with respect to the valve body in communication with an outside, wherein a proximal end portion of the outer needle has a tapered shape that is enlarged in diameter toward a proximal end, in a connecting portion between the outer needle and the passage forming member, an inner diameter dimension of the passage forming member is equal to or smaller than that of the outer needle.

According to the outer needle assembly,
the diameter-enlarged portion whose proximal end side
is increased in diameter is not formed at the connecting portion between the outer needle and the passage forming member. Therefore, at the connecting portion between the outer needle and the passage forming member in the bore of the outer needle assembly, a region which is not filled with the liquid introduced from the outer needle into the passage forming member is less prone to be formed, thereby suppress remaining of the air.

Besides, since the proximal end portion of the outer needle has a tapered shape whose diameter is enlarged toward the proximal end, the flow velocity of the liquid flowing through the outer needle decreases toward the proximal end. As a result, occurrence of turbulence due to the flow of the liquid is further suppressed at the proximal end of the outer needle, thereby preventing the air entrainment in the liquid.

A third example provides the outer needle assembly according to the second example, wherein in the connecting portion between the outer needle and the passage forming member, the inner diameter dimension of the passage forming member is equal to that of the outer needle.

According to the outer needle assembly,
the formation of a step is prevented at the connecting
portion between the outer needle and the passage forming member. Thus, occurrence of turbulence is further reduced in the liquid flowing through the bore of the outer needle assembly, thereby suppressing the air entrainment.

A fourth example provides the outer needle assembly according to any one of the first through third examples, wherein the passage forming member includes a flexible tube, and the flexible tube is connected to the outer needle.

According to the outer needle assembly,
in the outer needle assembly stuck and indwelled in a
blood vessel or the like, it is possible to easily connect the outer needle assembly to an external passage such as a dialysis circuit and an infusion line by bending the tube as needed, for example.

A fifth example provides the outer needle assembly according to the fourth example, wherein a distal end portion of the flexible tube includes a large-diameter tube part whose inner diameter dimension is increased, and a proximal end portion of the outer needle is fastened to the large-diameter tube part in an inserted state.

According to the outer needle assembly structured following the present example, by inserting and fixing the proximal end portion of the outer needle into the large-diameter tube part of the tube, the inner diameter dimension of the tube at the connecting portion between the outer needle and the tube can be easily made equal to or smaller than that of the outer needle.

A sixth example provides the outer needle assembly according to the fifth example, wherein the flexible tube includes a small-diameter tube part provided on the proximal end side with respect to the large-diameter tube part, the small-diameter tube part having an inner diameter dimension and an outer diameter dimension that are smaller than those of the large-diameter tube part.

According to the outer needle assembly, it is possible to prevent the passage forming member
from becoming thicker than necessary on the proximal end side with respect to the connecting portion between the outer needle and the passage forming member while preventing the inner diameter from suddenly becoming enlarged at the connecting portion between the outer needle and the passage forming member.

A seventh example provides the outer needle assembly according to any one of the first through sixth examples, wherein the passage forming member includes a flexible tube, and an inner circumferential surface ranging from a distal end of the outer needle to a proximal end of the flexible tube is not provided with a stepped diameter-enlarged portion.

According to the outer needle assembly,
a sudden enlargement of the inner diameter dimension
is avoided in a range from the distal end of the outer needle to the proximal end of the flexible tube. This will suppress sudden volume change in the internal space of the outer needle assembly, and entrainment and pool of the air in the portion of the said sudden volume change can be suppressed. The stepped diameter-enlarged portion is a portion where the passage cross-sectional area suddenly changes, and includes, for example, a stepped surface extending in the axis-perpendicular direction.

An eighth example provides a needle assembly comprising: a needle; a passage forming member connected to the needle; and a valve body arranged inside the passage forming member, a first internal space on a distal end side with respect to the valve body being longer than a second internal space on a proximal end side with respect to the valve body, wherein a ventilation path for discharging air in the first internal space to an outside communicates with the first internal space in a vicinity of the valve body, and the first internal space comprises: a puncture part formed in a portion of the needle, the portion puncturing a blood vessel; a tapered part formed on the proximal end side with respect to the puncture part, the tapered part having an inner diameter dimension which increases toward the proximal end side; and a small-diameter part formed on the proximal end side with respect to the tapered part, the small-diameter part having an inner diameter dimension which is smaller than or equal to that of a proximal end of the tapered part.

In the preceding first example, the outer needle and the inner needle are combined, and the effect of preventing remaining of air is exhibited in the bore of the outer needle assembly including the outer needle. The same effect is exhibited in the needle assembly including a single needle as well, and the effect of preventing remaining of air is exhibited in the bore of the needle assembly including the needle. It should be noted that the present example can be understood to be implementable by using the outer needle (48) as the needle of the present example in the practical example described later.

### EFFECT OF THE INVENTION

According to the present invention, it is possible to prevent air from remaining in the bore of the outer needle assembly.

### BRIEF DESCRIPTION OF THE DRAWINGS

FIG. 1 is a cross sectional view of an indwelling needle assembly according to a first practical embodiment of the present invention.
FIG. 2 is an enlarged cross sectional view of a part of the indwelling needle assembly shown in FIG. 1.
FIG. 3 is a cross sectional view of an outer needle assembly constituting the indwelling needle assembly shown in FIG. 2.
FIG. 4 is a cross sectional view showing a state in which an external passage is connected to the outer needle assembly shown in FIG. 3.
FIG. 5 is a cross sectional view of an outer needle assembly according to a second practical embodiment of the present invention.
FIG. 6 is a cross sectional view of an outer needle assembly according to a third practical embodiment of the present invention.
FIGS. 7A and 7B are photographs when a test was conducted to observe a flow mode of a liquid in an outer needle assembly with respect to the outer needle assembly of the conventional structure, where FIG. 7A shows a state before an inner needle was pulled out from an outer needle, and FIG. 7B shows a state in which the inner needle was pulled out from the outer needle.

### EMBODIMENTS FOR CARRYING OUT THE INVENTION

Hereinafter, practical embodiments of the present invention will be described in reference to the drawings.

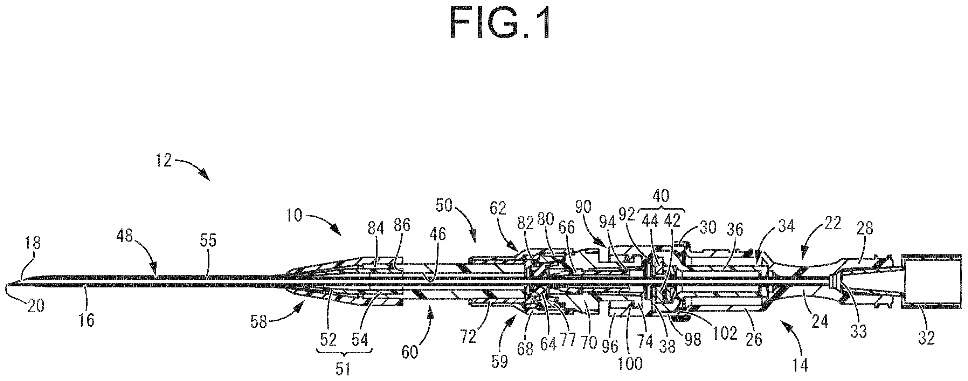

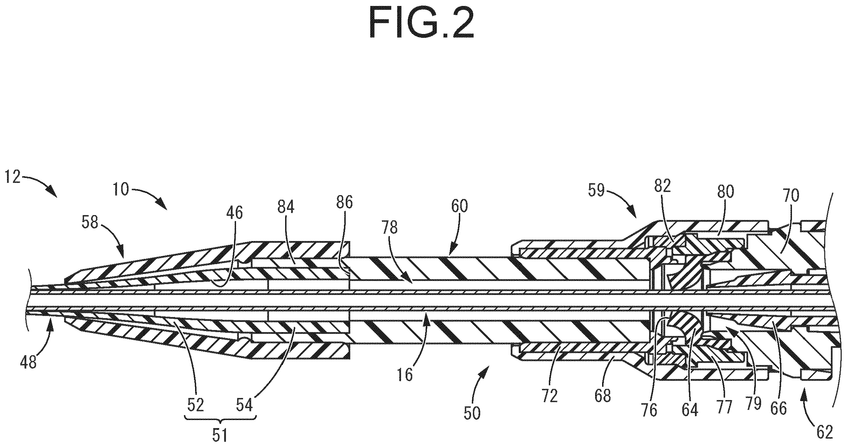

FIGS.1 and 2 show an indwelling needle assembly 12 including an outer needle assembly 10 as the first practical embodiment of the present invention. The indwelling needle assembly 12 has a structure in which an inner needle assembly 14 and the outer needle assembly 10 are combined. In the following description, as a general rule, the axial direction refers to the left-right direction in FIG. 1, with the left end in FIG. 1 indicating the distal end and the right end in FIG. 1 indicating the proximal end.

The inner needle assembly 14 includes an inner needle 16. The inner needle 16 is a hollow metal needle, and has a blade surface 18 having an inclined distal end surface, whereby an acute-angled needle tip 20 is formed. In FIG. 1, the blade surface 18 of the inner needle 16 is provided on the upper side in the drawing.

An inner needle hub 22 is provided on the proximal end side of the inner needle 16. The inner needle hub 22 has a pedestal part 24 to which the proximal end portion of the inner needle 16 is fixed in an inserted state. A protector accommodation part 26 is provided on the distal end side of the pedestal part 24, and a linking part 28 is provided on the proximal end side of the pedestal part 24.

The protector accommodation part 26 has a tubular shape. On the distal end side of the protector accommodation part 26, a restricting tube part 30, which has a diameter larger than that of the protector accommodation part 26 and has an approximately tubular shape, is provided. The outer peripheral surface of the protector accommodation part 26 may be provided with a slip stopper such as projections, recesses, and grains.

The linking part 28 has a tubular shape, to which an inner needle cap 32 is removably inserted and attached. The inner needle cap 32 has an approximately stepped cylindrical shape with a step provided in the axially middle portion thereof. A film-like filter 33 having properties of allowing passage of gas and preventing passage of liquid is provided at the distal end portion of the inner needle cap 32 so that the blood return through the inner needle 16 will not leak to the outside. By making the inner needle hub 22 and the inner needle cap 32 transparent or translucent, puncture of the blood vessel can be easily confirmed by the blood return (flashback).

A protector housing 34 is arranged on the radially inner side of the protector accommodation part 26. The protector housing 34 includes a tubular accommodation tube part 36 and a lid body 38 that closes off the opening on the distal end side of the accommodation tube part 36. Besides, the lid body 38 includes a plate-shaped part on the distal end side and a plate-shaped part on the proximal end side that are apart from each other in the axial direction, and a needle tip protector 40 is arranged between the plate-shaped parts.

The needle tip protector 40 comprises a shielding member 42 and a fixing member 44 housed in the protector housing 34. The shielding member 42 and the fixing member 44 are arranged apart from each other with the inner needle 16 interposed therebetween in the axis-perpendicular direction. One of the shielding member 42 and the fixing member 44 is a magnet, and the other is a magnet or a ferromagnet, and a magnetic attractive force that attracts each other acts between the shielding member 42 and the fixing member 44. In the present practical embodiment, the shielding member 42 is formed of a ferromagnetic material such as iron, and the fixing member 44 is a permanent magnet. The fixing member 44 is fixed to the distal end part of the accommodation tube part 36. The shielding member 42 is displaceable in the direction of approach with respect to the fixing member 44. With the inner needle 16 inserted between the shielding member 42 and the fixing member 44, movement of the shielding member 42 toward the fixing member 44 is prevented by the inner needle 16. Then, by pulling out the inner needle 16 to the proximal end side with respect to the shielding member 42, the shielding member 42 is allowed to move toward the fixing member 44 by the magnetic attractive force, and the shielding member 42 moves to a position where the shielding member 42 covers the needle tip 20 of the inner needle 16, so that movement of the inner needle 16 toward the distal end is prevented by the shielding member 42.

The inner needle 16 is inserted through the outer needle assembly 10 in a removable mode. The outer needle assembly 10 is a tubular body penetrated by a bore 46 that serves as an internal space in the axial direction. The outer needle assembly 10 has an outer needle 48 and an outer needle hub 50 that serves as a passage forming member and is connected to the proximal end side of the outer needle 48 in a communicating state.

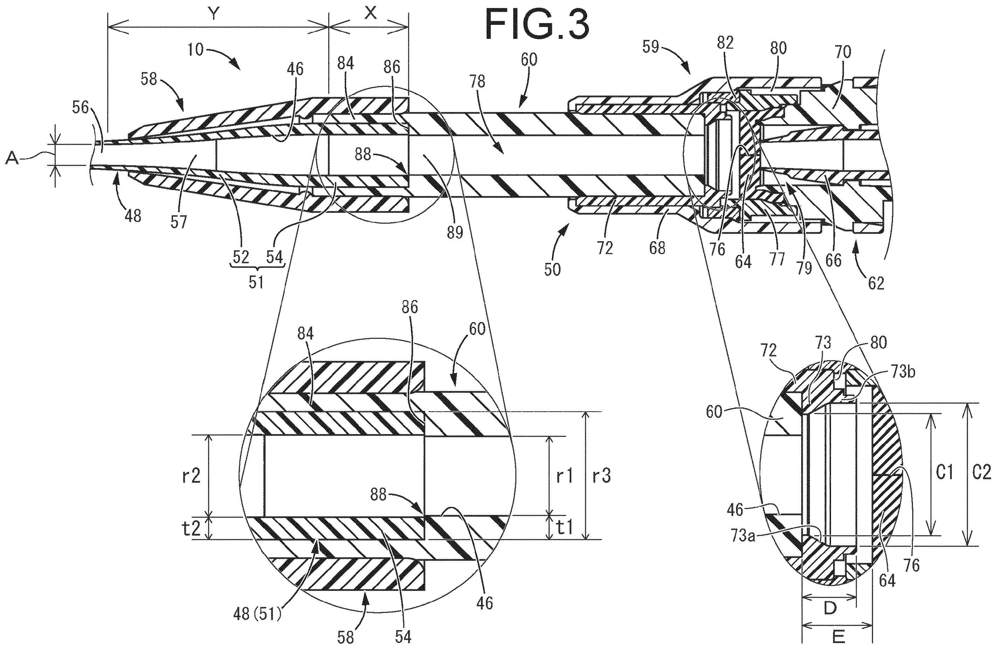

The outer needle 48 has a hollow small-diameter tube shape made of synthetic resin or the like. The outer peripheral surface of the outer needle 48 has a tapered shape whose distal end gradually decreases in diameter. As shown in FIG. 3, a proximal end portion 51 of the outer needle 48 includes a tapered first portion 52 which is enlarged in diameter toward the proximal end so as to have an increased diameter, and a second portion 54 which is positioned on the proximal end side with respect to the first portion 52 and extends with approximately constant inner and outer diameter dimensions. That is, the outer needle 48 of the present practical embodiment is a specific-shaped tube of taper-integral type which is integrally equipped with the tapered first portion 52. The proximal end portion 51 of the outer needle 48 has a diameter larger than that of a distal end portion 55 of the outer needle 48 by having the tapered first portion 52. The proximal end portion 51 of the outer needle 48 in the present practical embodiment gradually becomes thicker toward the proximal end in the first portion 52, and is thicker than the distal end portion 55 of the outer needle 48. The bore of the outer needle 48 includes a puncture part 56 extending with an approximately constant diameter in the distal end portion 55 that punctures a blood vessel, and a tapered part 57 whose inner diameter dimension increases toward the proximal end side in the first portion 52 of the proximal end portion 51 that is located on the proximal end side with respect to the puncture part 56. The inner circumferential surface of the outer needle 48 is formed of a curved surface that continues smoothly without forming corners such as steps in the axial direction and in the circumferential direction. It would be acceptable as long as the tapered first portion 52 (the tapered part 57) is enlarged in diameter toward the proximal end overall, and its taper angle may be constant or may change in the axial direction.

Although it depends on the thickness of the inner needle 16 inserted through the outer needle 48, the axial length Y of the tapered first portion 52 (see FIG. 3) is relatively longer than the axial length X of the second portion 54 extending approximately straight in the axial direction (see FIG. 3) so as to be, for example, 1.5X ≤ Y, and 2X < Y in the present practical embodiment. Besides, although the taper angle α of the first portion 52 is not limited, the taper angle α of the first portion 52 is set relatively moderate because the length dimension Y of the tapered first portion 52 is relatively long. The taper angle α of the first portion 52 is an inclination angle of a straight line connecting the proximal end of the distal end portion 55 and the distal end of the second portion 54 located on the opposite sides of the first portion 52, with respect to the axial direction. In this way, by setting the taper angle α of the first portion 52 relatively moderate, a sudden volume change in the internal space of the outer needle 48 is suppressed. Here, the axial length Y of the first portion 52 means the axial length between the distal end portion 55 and the second portion 54 of the outer needle 48 each extending with approximately constant inner and outer diameter dimensions.

The outer needle hub 50 has a tubular shape overall, and has a structure in which a needle joining member 58 and a passage connecting member 59 are linked by a tube 60.

The needle joining member 58 is made of a rigid synthetic resin. The needle joining member 58 has a tapered tube shape decreasing in diameter toward the distal end. The proximal end portion 51 of the outer needle 48 is inserted in the radial inside of the needle joining member 58.

The passage connecting member 59 has a tubular structure including a connecting part at its proximal end connected to an external passage 104, which will be described later, and in the present practical embodiment, the passage connecting member 59 includes a tubular valve housing 62 that houses a valve body 64 and a pusher 66.

The valve housing 62 includes a tubular cover member 68 and a tubular pusher guide 70, and is constituted by the distal end portion of the pusher guide 70 being inserted into the proximal end portion of the cover member 68 so as to be linked to each other in the axial direction.

A tube linking member 72 is attached to the cover member 68 in an inserted state. The tube linking member 72 is overlapped with and fixed to the inner circumferential surface of the distal end portion of the cover member 68. The proximal end portion of the tube linking member 72 is situated in opposition to the cover member 68 at a position away from the cover member 68 to the radial inside. The proximal end portion of the tube linking member 72 is provided with an annular tube fixing part 73 projecting radially inward. The distal side end face of the tube fixing part 73 is an annular flat surface, with which the proximal end surface of the tube 60 is in contact, while the inner circumferential surface of the tube fixing part 73 is a tapered surface 73a that is gradually enlarged in diameter toward the proximal end side. At the proximal end of the tapered surface 73a, an approximately tubular proximal end tube part 73b is provided so as to project to the proximal end side with a constant inner diameter dimension from the portion whose inner diameter dimension is the largest.

That is, the minimum inner diameter dimension C1 (see FIG. 3) at the tapered surface 73a of the tube fixing part 73 is larger than the inner diameter dimension r1 of the tube 60 described later, and the radially inner end of the proximal end of the tube 60 projects to the radially inner side with respect to the radially inner end of the tube fixing part 73. Further, the minimum inner diameter dimension C1 at the tapered surface 73a is smaller than the outer diameter dimension of the tube 60. Moreover, the inner diameter dimension C2 (see FIG. 3) of the proximal end tube part 73b of the tube fixing part 73 is larger than the minimum inner diameter dimension C1 of the tapered surface 73a. That is, in the opening portion on the proximal end side of the tube 60, by providing the tube fixing part 73 having the tapered surface 73a, the inner diameter dimension of a first internal space 78, which will be described later, increases stepwise or gradually toward a ventilation path 80. The axial dimension D (see FIG. 3) of the tube fixing part 73 including the proximal end tube part 73b is set relatively small.

In the pusher guide 70, the proximal end portion extends in the axial direction with an approximately constant cross-sectional shape, and a male screw part 74 projecting radially outward is integrally formed at the proximal end (see FIG. 1).

As shown in FIG. 3, the valve body 64 has an approximately circular disk shape overall, and is formed of an elastic body such as a resin elastomer and rubber. The valve body 64 has a radial slit 76 formed in the central portion of the circular disk shape, and a slit 76 is configured to be opened and closed by elastic deformation of the central portion. The outer peripheral portion of the valve body 64 is axially clasped between the distal end of the pusher guide 70 and a tubular valve support member 77 arranged in the radial inside of the cover member 68. With this arrangement, the valve body 64 is supported by the valve housing 62, and the bore of the valve housing 62 is blocked by the valve body 64. By the bore of the valve housing 62 being blocked by the valve body 64, the bore 46 of the outer needle assembly 10 is divided into a distal end side and a proximal end side with respect to the valve body 64. Accordingly, in the bore 46, the distal end side with respect to the valve body 64 constitutes a first internal space 78, and the proximal end side with respect to the valve body 64 constitutes a second internal space 79. The first internal space 78 is longer than the second internal space 79 in the axial direction.

Besides, with the valve body 64 housed in the valve housing 62, partly because the axial length D of the tube fixing part 73 that supports the tube 60 from the proximal end side is relatively small, the proximal end surface of the tube 60 and the distal end surface of the valve body 64 are opposed so as to be relatively close to each other in the axial direction. In particular, the tube fixing part 73 includes the proximal end tube part 73b projecting to the proximal end side, and the axial distance between the proximal end tube part 73b and the distal end surface of the valve body 64 is smaller than the distance between the proximal end surface of the tube 60 and the distal end surface of the valve body 64. Then, as will be described later, the first internal space 78 of the outer needle assembly 10 communicates with the ventilation path 80 through the gap between the proximal end tube part 73b and the distal end surface of the valve body 64 opposed to each other. The proximal end of the tube 60 and the valve body 64 are relatively close to each other, and the valve body 64 readily guides the gas outward in the circumferential direction, making it difficult for air bubbles to remain.

The valve support member 77 is arranged at a position slightly away from the cover member 68 to the radial inside, and a gap is provided radially between the valve support member 77 and the cover member 68. The ventilation path 80 that places the bore 46 of the outer needle assembly 10 in communication with the external space is constituted by including the gap between the valve support member 77 and the cover member 68. One end of the ventilation path 80 communicates with the first internal space 78 of the outer needle assembly 10 passing between the tube linking member 72 and the valve support member 77, while the other end thereof communicates with the external space passing between the cover member 68 and the pusher guide 70. With this configuration, the distal end side of the bore 46 of the outer needle assembly 10 with respect to the valve body 64 (namely, the first internal space 78) communicates with the external space through the ventilation path 80. Besides, it is desirable that the ventilation path 80 have a small cross-sectional area to allow the flow of gas and limit the flow of liquid. Further, the ventilation path 80 of the present practical embodiment has a large cross-sectional area in the middle portion thereof. Thus, if blood enters the ventilation path 80, the blood will be stored in its middle portion and it will be difficult for the blood to leak from the ventilation path 80 to the external space. Note that the configuration of the ventilation path is not limited to the mode of the present practical embodiment. The ventilation path may be provided on the outer surface side of the valve body, for example, so that the gas moves to the proximal end side through the outer surface side of the valve body and is discharged to the external space.

Therefore, in the present practical embodiment, the ventilation path 80 is provided on the radially outer side with respect to the valve body 64, and the opening of the ventilation path 80 on the first internal space 78 side is provided on the radially outer side with respect to the tube 60 and the tube fixing part 73. Besides, the inner diameter dimension of the proximal end portion of the tube 60 is increased in a stepwise manner by the tube fixing part 73. Thus, in other words, at the proximal end portion of the tube 60, the inner diameter dimension is increased from the proximal end side opening of the tube 60 toward the ventilation path 80.

A ventilation filter 82 is arranged in the ventilation path 80. The ventilation filter 82 is a filter that allows passage of gas and limits passage of liquid such as blood, and has a cylindrical or annular shape. The ventilation filter 82 is arranged radially between the tube linking member 72 and the valve support member 77 on one side, and the cover member 68 on the other, so as to be held in a compressed state in the radial direction. Since the ventilation filter 82 is arranged on the ventilation path 80, with the bore 46 of the outer needle assembly 10 filled with blood, the infiltration of the blood into the ventilation path 80 is suppressed by the ventilation filter 82, thereby preventing leakage of the blood through the ventilation path 80 to the outside.

The pusher 66 has a tubular shape and is inserted into the radial inside of the pusher guide 70. The pusher 66 is configured such that the proximal end portion has an approximately constant outer diameter dimension, and the outer peripheral surface of the distal end portion has a tapered shape that decreases in diameter toward the distal end side.

The tube 60 connecting the needle joining member 58 and the passage connecting member 59 is a flexible soft tube made of a resin elastomer, rubber, or the like. The tube 60 can be deformed to curve, and its cross-sectional shape is allowed to change. By providing the tube 60, for example, it becomes easy to connect the external passage 104 to the outer needle hub 50 with the needle joining member 58 fixed to the skin. The distal end portion of the tube 60 is fastened to the needle joining member 58, while the proximal end portion thereof is inserted into and fastened to the radial inside of the tube linking member 72, which is attached to the passage connecting member 59. With this arrangement, the needle joining member 58 and the passage connecting member 59 are connected by the tube 60, and the bore of the passage connecting member 59 communicates with the bore of the tube 60. The middle portion of the tube 60 located between the distal end portion and the proximal end portion thereof is away from both the needle joining member 58 and the passage connecting member 59, and is allowed to deform to bend, collapse, and the like.

The proximal end portion 51 of the outer needle 48 is inserted and connected to the distal end portion of the tube 60. In the present practical embodiment, the distal end portion of the tube 60 is clasped and fastened radially between the proximal end portion 51 of the outer needle 48 and the needle joining member 58, and the outer needle hub 50 including the needle joining member 58 and the tube 60 is connected to the proximal end side of the outer needle 48. The method of fastening the needle joining member 58, the proximal end portion 51 of the outer needle 48, and the distal end portion of the tube 60 is not limited, but for example, the proximal end portion 51 of the outer needle 48 is inserted in a press-fitted state into a large-diameter tube part 84 of the tube 60, and is adhered as needed. Besides, a projection on the inner surface of the needle joining member 58 may be used to position the insertion end of the tube 60 into the needle joining member 58, and to hold the large-diameter tube part 84 by clamping. The bore of the outer needle 48 and the bore of the outer needle hub 50 communicate with each other, and the bore 46 of the outer needle assembly 10 includes the bore of the outer needle 48 and the bore of the outer needle hub 50.

The distal end portion of the tube 60, which is fastened to outer needle 48, constitutes the large-diameter tube part 84 whose inner diameter dimension is larger than that of the portion located away from the outer needle 48 to the proximal end side. Since the outer diameter dimension of the tube 60 is approximately constant in the axial direction, the tube 60 is thin-walled in the large-diameter tube part 84. The tube 60 includes an annular stepped surface 86 that connects the inner circumferential surface of the large-diameter tube part 84 and the inner circumferential surface of the portion of the tube 60 that is away from the large-diameter tube part 84 to the proximal end side. The stepped surface 86 is provided on the inner circumferential portion of the tube 60 and extends in the axis-perpendicular direction. The tube 60 has an approximately constant inner diameter dimension r1 (described later) on the proximal end side of the stepped surface 86.

The second portion 54 constituting the proximal end portion 51 of the outer needle 48 is inserted into the radial inside of the large-diameter tube part 84, and is fastened to the tube 60 in an inserted state. In other words, the large-diameter tube part 84, which is the distal end portion of the tube 60, is held in a state of being radially clasped between the second portion 54 of the outer needle 48 and the needle joining member 58. The outer peripheral surface of the second portion 54 of the outer needle 48 is overlapped with the inner circumferential surface of the large-diameter tube part 84 of the tube 60 in a state of contact. The proximal end surface of the outer needle 48 is abutted against the stepped surface 86 of the tube 60 in the axial direction, so that the outer needle 48 and the tube 60 are mutually positioned in the axial direction. That is, in the present practical embodiment, the outer needle 48 and the tube 60 are in direct contact with each other. The inner circumference of the outer needle 48 is not provided with an outer needle fixing member or the like for pressing the outer needle 48 against the large-diameter tube part 84 to fix thereto. Thus, it is possible to avoid formation of any step on the inner circumference of the outer needle 48 caused by arranging the said outer needle fixing member, thereby preventing entrainment of air in the blood or the like due to the step. Further, in the present practical embodiment, by setting the taper angle α of the tapered part 57 of the outer needle 48 to be relatively moderate, it is also possible to prevent air from being entrained or pooling due to a sudden volume change in the internal space of the outer needle 48. In particular, if the taper angle α is set to 30 degrees or more, or 45 degrees or more, the shape may be approximately stepped, and there is concern about entrainment, pool or the like of the air.

In a connecting portion 88 between the outer needle 48 and the tube 60, the inner diameter dimension r1 of the tube 60 is set to be equal to or smaller than the inner diameter dimension r2 of the outer needle 48 (r1 ≤ r2). In other words, the radial width dimension t1 of the stepped surface 86 is equal to or greater than the thickness dimension t2 at the proximal end of the outer needle 48 (t1 ≥ t2). Here, the radial width dimension t1 of the stepped surface 86 is obtained by subtracting the inner diameter dimension r1 of the tube 60 at the connecting portion 88 between the outer needle 48 and the tube 60 from the inner diameter dimension r3 of the proximal end of the large-diameter tube part 84 in the tube 60, then dividing the obtained dimension by 2 (t1 = (r3-r1) / 2). In the present practical embodiment, in the connecting portion 88 between the outer needle 48 and the tube 60, the inner diameter dimension r1 of the tube 60 is smaller than the inner diameter dimension r2 of the proximal end of the outer needle 48 to the extent that can be regarded as being substantially equal. In preferred practice, the inner diameter dimension r1 of the tube 60 is 2.6 mm or smaller, and more preferably the inner diameter dimension r1 of the tube 60 is 2.5 mm or smaller. Further, in preferred practice, the inner diameter dimension r2 of the outer needle 48 is 2.5 mm ≤ r2 ≤ 3.3 mm. More preferably, the inner diameter dimension r2 of the outer needle 48 is set to, for example, 2.9 mm, which is larger than the inner diameter dimension r1 of the tube 60. Besides, the inner diameter dimension of the tube 60 is approximately constant from the connecting portion 88 between the outer needle 48 and the tube 60 to the proximal end of the tube 60. Further, the inner diameter dimension of the outer needle hub 50 is equal to or smaller than the inner diameter dimension r2 of the proximal end of the outer needle 48 from the connecting portion 88 between the outer needle 48 and the tube 60 to the proximal end of the tube 60.

Additionally, as described above, the inner diameter dimension A (see FIG. 3) of the distal end portion 55 (the puncture part 56) of the outer needle 48 is equal to or smaller than the inner diameter dimension of the tapered part 57 of the outer needle 48, and is smaller than the inner diameter dimension r1 of the tube 60 and the inner diameter dimension r2 of the proximal end of the outer needle 48 (A < r1 ≤ r2). With this configuration, it is possible to achieve a sufficient flow amount and to make it difficult for the air bubbles to remain in a compatible manner.

The inner diameter dimension A of the puncture part 56 in the outer needle 48 is, although it depends on the thickness or the like of the inner needle 16 inserted through the outer needle 48, preferably such that (the inner diameter dimension r1 of the tube 60) < (1.5 × A), for example. This configuration makes it possible to reduce the change in the cross-sectional area of the internal space of the outer needle 48, thereby making it difficult for the air bubbles to be entrained. Besides, it is preferable that (6 × A) < (the axial length Y of the tapered part 57). With this configuration, it is possible to reliably obtain a sufficiently large axial length Y of the tapered part 57, so that the tapered part 57 can be formed into a shape that is moderately enlarged in diameter, thereby suppressing remaining of the air bubbles. Moreover, the inner diameter dimension r1 of the tube 60 is preferably such that (the inner diameter dimension C2 of the proximal end tube part 73b) < (2 × r1), for example. Further, the inner diameter dimension r1 of the tube 60 is preferably such that (the axial length E between the valve body 64 and the tube 60) < (2 × r1), for example. With this configuration, the valve body 64 and the tube 60 can be arranged so as to be close to each other. In the present practical embodiment, the axial length E between the valve body 64 and the tube 60 is smaller than the inner diameter dimension r1 of the tube 60. Furthermore, the inner diameter dimension r1 of the tube 60 is preferably such that (the axial length D of the tube fixing part 73) < r1, for example. This also makes it possible to arrange the valve body 64 and the tube 60 in a relatively close state. In the first internal space 78, the ventilation path 80 opens on the front surface of the valve body 64. Additionally, since the inner diameter dimension r1 of the tube 60 and the inner diameter dimension r2 of the proximal end of the outer needle 48 can be regarded as being substantially equal, the same relationship as described above is considered to be exist with respect to the inner diameter dimension r2 of the proximal end of the outer needle 48. That is, the inner diameter dimension r2 of the proximal end of the outer needle 48 is preferably such that (the inner diameter dimension r2 of the proximal end of the outer needle 48) < (1.5 × A). Besides, the inner diameter dimension r2 of the proximal end of the outer needle 48 is preferably such that (the inner diameter dimension C2 of the proximal end tube part 73b) < (2 × r2), for example. Moreover, the inner diameter dimension r2 of the proximal end of the outer needle 48 is preferably such that (the axial length E between the valve body 64 and the tube 60) < (2 × r2), for example. Furthermore, the inner diameter dimension r2 of the proximal end of the outer needle 48 is preferably such that (the axial length D of the tube fixing part 73) < r2, for example.

The connecting portion 88 between the outer needle 48 and the tube 60 is the portion that constitutes a boundary between the outer needle 48 and the tube 60 on the inner surface of the internal passage of the outer needle assembly 10. The connecting portion 88 between the outer needle 48 and the tube 60 in the present practical embodiment is provided at the portion where the proximal end surface of the outer needle 48 and the stepped surface 86 of the tube 60 are located in the axial direction. The connecting portion 88 between the outer needle 48 and the tube 60 may include a portion of the tube 60 adjacent to the large-diameter tube part 84 on the proximal end side. However, the focus of attention in the present invention is the internal passage of the outer needle assembly 10 (the bore 46) and the inner surface of the inner passage. Thus, it should be understood that the tapered shape of the outer needle 48 and the connecting portion 88 between the outer needle 48 and the tube 60, for example, refer to those of the passage inner surface (the inner circumferential surface) of the internal passage.

In the bore of the tube 60, the proximal end side with respect to the large-diameter tube part 84 is provided on the proximal end side with respect to the proximal end of the tapered part 57 of the outer needle 48, and constitutes a small-diameter part 89 that has a smaller diameter than that of the proximal end of the tapered part 57. The small-diameter part 89 is axially continuous from the connecting portion 88 between the outer needle 48 and the tube 60 to the proximal end of the tube 60. The small-diameter part 89 extends with an approximately constant diameter dimension, and the inner circumferential surface of the tube 60 has a cylindrical shape at the portion constituting the small-diameter part 89. The small-diameter part 89 has an axial length longer than that of the tapered part 57 of the outer needle 48. The first internal space 78 located on the distal end side with respect to the valve body 64 in the bore 46 of the outer needle assembly 10 includes the puncture part 56 and the tapered part 57 constituting the bore of the outer needle 48, and the small-diameter part 89 constituting the bore of the tube 60.

The inner needle 16 is inserted from the proximal end side of the bore 46 of the outer needle assembly 10 including the bore of the outer needle 48 and the bore of the outer needle hub 50. As shown in FIGS. 1 and 2, the inner needle 16 pushes open the slit 76 of the valve body 64 arranged in the bore 46 of the outer needle assembly 10 and penetrates the slit 76.

The inner needle assembly 14 provided with the inner needle 16 and the outer needle assembly 10 provided with the outer needle 48 are connected by a connector cap 90. The connector cap 90 includes a bottom wall 92 extending in the axis-perpendicular direction. The bottom wall 92 has an annular disk shape, and a pipe-shaped projection 94 projecting to the distal end side is integrally formed with its radially inner end. At the outer peripheral end of the bottom wall 92, a peripheral wall 96 extending to the distal end side and a pair of elastic pieces 98, 98 extending to the proximal end side are integrally formed.

A female thread 100 is provided on the inner circumferential surface of the tubular peripheral wall 96. Then, the proximal end portion of the pusher guide 70 constituting the outer needle hub 50 is inserted into the radial inside of the peripheral wall 96, and the male screw part 74 of the pusher guide 70 and the female thread 100 of the peripheral wall 96 are screwed together, whereby the connector cap 90 is attached to the proximal end portion of the outer needle hub 50.

With the inner needle assembly 14 and the outer needle assembly 10 assembled, the restricting tube part 30 of the inner needle hub 22 is externally fitted onto the connector cap 90. With this arrangement, the deformation of the elastic pieces 98, 98 to the radially outer side is restricted by the restricting tube part 30, and the connection between the connector cap 90 and the protector housing 34 by pair of locking projections 102, 102 is stably maintained.

Regarding the indwelling needle assembly 12 constructed as described above, the inner needle 16 will be stuck into a blood vessel of a patient's arm or the like (not shown). A health care worker (a user) who punctures a blood vessel holds the inner needle hub 22 with his/her fingers and sticks the inner needle 16 into the patient's arm or the like. After the inner needle 16 and the outer needle 48 are stuck into the patient's blood vessel, the inner needle 16 is removed from the outer needle 48, so that the outer needle assembly 10 is indwelled with the outer needle 48 inserted in the patient's blood vessel.

The bore 46 of the outer needle assembly 10 is filled with air before the inner needle 16 and the outer needle 48 are stuck. Then, when the inner needle 16 is removed from the outer needle 48, blood flows into the bore 46 of the outer needle assembly 10 from the distal end opening of the outer needle 48. Here, in the connecting portion 88 between the outer needle 48 and the tube 60 on the wall inner surface of the bore 46 of the outer needle assembly 10, the inner diameter dimension r1 of the tube 60 is equal to or smaller than the inner diameter dimension r2 of the proximal end of the outer needle 48. Therefore, the wall inner surface of the bore 46 of the outer needle assembly 10 is not provided with a stepped diameter-enlarged portion at the connecting portion 88 between the outer needle 48 and the tube 60. Therefore, a region of remaining air that is not filled with the blood is less prone to be formed in the vicinity of the connecting portion 88 between the outer needle 48 and the tube 60, so that the bore 46 of the outer needle assembly 10 can be filled with the blood. It is preferable that the inner diameter dimension r1 of the tube 60 is smaller than the inner diameter dimension r2 of the proximal end of the outer needle 48 because the air bubbles are less likely to remain.

With the outer needle stuck in the blood vessel, there may be a case where the distal end of the outer needle assembly is tilted such that its distal end is positioned above its proximal end. In that case, if a diameter-enlarged portion of stepped shape whose proximal end side is enlarged in diameter is formed in the connecting portion between the outer needle and the tube in the bore of the outer needle assembly, it is difficult for the blood to flow into the said diameter-enlarged portion, and the air is likely to remain. With the outer needle assembly 10 according to the present practical embodiment, no stepped diameter-enlarged portion is provided at the connecting portion 88 between the outer needle 48 and the tube 60 in the bore 46 of the outer needle assembly 10. Thus, the air is efficiently pushed out to the proximal end side by the blood, so that the air will be discharged to the outside from the ventilation path 80 without remaining in the bore 46. For example, in the case of indwelling a plurality of outer needle assemblies such as an indwelling needle on the blood removal side and an indwelling needle on the blood return side connected to the dialysis circuit, at least one outer needle assembly may be oriented such that its distal end is positioned on the upper side.

Besides, in the connecting portion 88 between the outer needle 48 and the tube 60, no stepped diameter-enlarged portion is formed on the wall inner surface of the bore 46 of the outer needle assembly 10, thereby reducing generation of turbulence due to a sudden expansion of the cross-sectional area. Therefore, the air entrainment due to turbulence is reduced, and the remaining of the air is suppressed in the bore 46 of the outer needle assembly 10. In the present practical embodiment in particular, from the distal end of the outer needle 48 to the proximal end of the tube 60, a stepped diameter-enlarged portion or a portion whose taper angle suddenly increases is not provided on the inner circumferential surface of the outer needle assembly 10. This configuration suppresses sudden volume change in the bore 46, thereby further preventing entrainment, pool or the like of the air. It is preferable that the bore 46 of the outer needle assembly 10 of the present practical embodiment is not provided with a stepped diameter-enlarged portion that exceeds the wall thickness of the outer needle.

The proximal end portion 51 of the outer needle 48 includes the first portion 52 whose inner circumferential surface has a moderately tapered shape, and the inner diameter dimension of the proximal end portion 51 increases toward the proximal end. With this configuration, at the proximal end of the outer needle 48 located at the connecting portion 88 between the outer needle 48 and the tube 60, the flow velocity of blood is smaller than that at the distal end of the outer needle 48. Thus, the air is more reliably pushed out to the proximal end side by the blood, and the turbulence is easily prevented, thereby more effectively preventing the air from remaining in the bore 46.

The connecting portion 88 between the outer needle 48 and the tube 60 is the portion where the proximal end portion 51 of the outer needle 48 is enlarged in diameter in a tapered shape and the flow velocity suddenly decreases, and in addition, a stepped diameter-enlarged portion is likely to be formed. Thus, in addition to the fact that blood is likely to be divided by air in the passage, the connecting portion 88 is located away from the ventilation path 80 toward the distal end side. Therefore, when the air remains or is entrained in the connecting portion 88 between the outer needle 48 and the tube 60 due to the diameter enlargement, the remaining air finds it difficult to reach the ventilation path 80 and is likely to remain in the bore 46 of the outer needle assembly 10. Accordingly, the present embodiment prevents the sudden diameter enlargement of the connecting portion 88 between the outer needle 48 and the tube 60 toward the proximal end of the bore 46, and suppresses the remaining or entrainment of the air in the connecting portion 88 between the outer needle 48 and the tube 60. This arrangement makes it possible to prevent the air from remaining in the bore 46 of the outer needle assembly 10 and fill the bore 46 with the blood. The bore 46 of the outer needle assembly 10 of the present practical embodiment is enlarged in diameter in a stepped shape at the proximal end of the tube 60. However, the said diameter-enlarged portion is away from the proximal end portion 51 of the outer needle 48, which is enlarged in diameter in a tapered shape, and is provided at the position closer to the ventilation path 80 than the connecting portion 88 between the outer needle 48 and the tube 60. Thus, the said diameter-enlarged portion is less likely to cause remaining of the air in the bore 46. In the present practical embodiment in particular, the ventilation path 80 is provided on the radially outer side of the tube 60, and the bore 46 of the outer needle assembly 10 is enlarged in diameter stepwise or gradually at the proximal end of the tube 60 so as to spread in the direction toward the ventilation path 80.

In the first internal space 78 of the outer needle assembly 10, the inner diameter dimension of the small-diameter part 89 formed on the proximal end side with respect to the tapered part 57 is smaller than the inner diameter dimension of the proximal end of the tapered part 57. With this configuration, when the blood flows from the proximal end of the tapered part 57 having a large passage cross sectional area to the small-diameter part 89 having a small passage cross sectional area, the blood flows into the entire passage cross section of the small-diameter part 89 without flowing in a part of the passage cross section of the small-diameter part 89 in advance, so that the blood is less likely to wrap around to the proximal end side with the air left. Therefore, the ventilation path 80 that opens in the vicinity of the valve body 64 on the proximal end side with respect to the small-diameter part 89 will not be blocked by the preceding blood, and the air is effectively discharged through the ventilation path 80, thereby preventing the air from remaining in the first internal space 78 at the time of blood introduction.

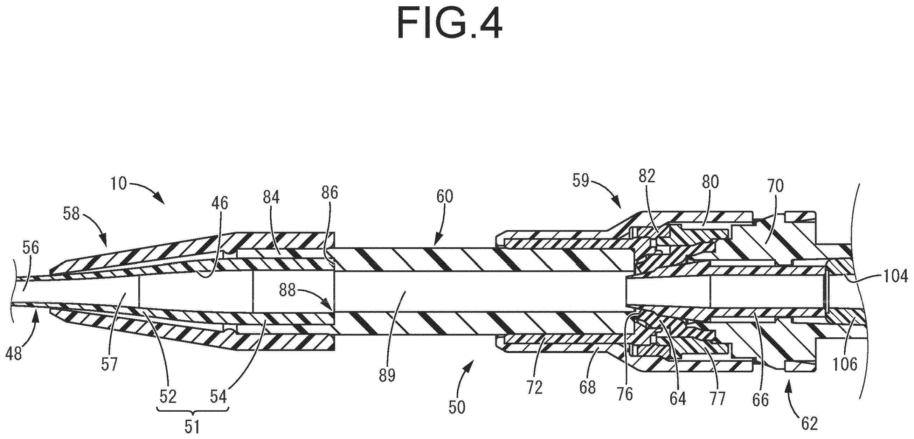

In the outer needle assembly 10 separated from the inner needle 16 and indwelled, the connector cap 90 is removed from the outer needle hub 50. Then, as shown in FIG. 4, a male connector 106 constituting an external passage 104 is inserted into the pusher guide 70 of the outer needle hub 50 from the proximal end side. Besides, a lock part (not shown) provided to the male connector 106 constituting the external passage 104 is screwed to the male screw part 74 provided to the passage connecting member 59 of the outer needle hub 50. With this arrangement, the outer needle assembly 10 and the external passage 104 are connected, and the bore 46 of the outer needle assembly 10 communicates with the external passage 104. In this way, the male screw part 74 of the outer needle hub 50 is used for both the connection of the connector cap 90 to the outer needle hub 50 and the connection of the external passage 104 to the outer needle hub 50. The male connector 106 does not have to have the lock part (not shown), and it would also be possible to connect the male connector 106 and the pusher guide 70 by the male connector 106 being fitted into the proximal end of the pusher guide 70.

When the male connector 106 is connected to the outer needle hub 50, the pusher 66 is pushed to the distal end side by the male connector 106. The pushed pusher 66 is pressed against the valve body 64 to deform the valve body 64, so that the slit 76 of the valve body 64 is opened. By so doing, the patient's blood vessel and the external passage 104 are connected to each other through the bore 46 of the outer needle assembly 10, and treatment such as hemodialysis, blood collection, and administration of a drug solution can be performed.

With the external passage 104 connected to the outer needle assembly 10 as shown in FIG. 4, the valve body 64 pushed open by the pusher 66 covers the opening of the ventilation path 80 on the bore 46 side, and the ventilation path 80 is blocked by the valve body 64. By so doing, the valve body 64 prevents the leakage of the blood, the drug solution, and the like through the ventilation path 80 to the outside. However, blocking of the ventilation path 80 by the valve body 64 is not essential.

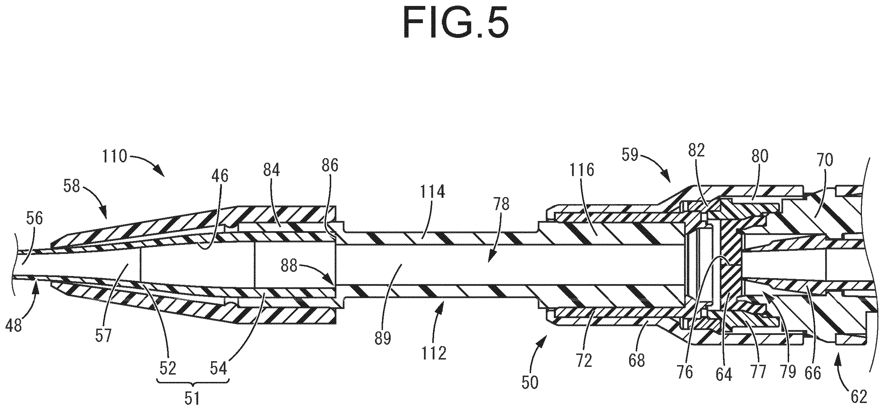

FIG. 5 shows an outer needle assembly 110 as a second practical embodiment of the present invention. The outer needle assembly 110 includes a tube 112. In the following description, components and parts that are substantially identical with those in the first practical embodiment will be assigned like symbols and not described in any detail.

The tube 112 has a structure in which a small-diameter tube part 114 is continuously provided on the proximal end side with respect to the large-diameter tube part 84 constituting the distal end portion. The small-diameter tube part 114 has an approximately cylindrical shape, and the inner diameter dimension and the outer diameter dimension are approximately constant in the axial direction. Both the inner diameter dimension and the outer diameter dimension of the small-diameter tube part 114 are smaller than those of the large-diameter tube part 84. The small-diameter tube part 114 of the present practical embodiment has approximately the same thickness dimension as the large-diameter tube part 84, but the small-diameter tube part 114 may be thicker or thinner than the large-diameter tube part 84.

A thick-walled linking part 116 is provided on the proximal end side of the small-diameter tube part 114. The thick-walled linking part 116 has the outer diameter dimension that is approximately equal to that of the large-diameter tube part 84, and the inner diameter dimension that is approximately equal to that of the small-diameter tube part 114. The thick-walled linking part 116 projects radially outward further than the small-diameter tube part 114 to be thick-walled. The outer diameter dimension of the tube 112 is made smaller in the small-diameter tube part 114, which is the axially middle portion, than in the large-diameter tube part 84 and the thick-walled linking part 116, which are the opposite end portions.

With the outer needle assembly 110 constructed according to the present practical embodiment, similarly to the first practical embodiment, in the connecting portion 88 between the outer needle 48 and the tube 112, the tube 112 side is not enlarged in diameter. Thus, when blood flows in, air is prevented from remaining in the bore 46 of the outer needle assembly 110.

Additionally, the tube 112 is configured such that the small-diameter tube part 114, which is provided on the proximal end side with respect to the portion fastened to the outer needle 48, is smaller in diameter than the large-diameter tube part 84 fastened to the outer needle 48. Therefore, the forming material can be saved by thinning the small-diameter tube part 114 while making the inner diameter dimension at the small-diameter tube part 114 of the tube 112 equal to or smaller than the inner diameter dimension of the proximal end of the outer needle 48.

Since the tube 112 is thin-walled in the small-diameter tube part 114, the elasticity of the small-diameter tube part 114 can be made small, thereby making the small-diameter tube part 114 easily deformable. Therefore, it is possible to facilitate the connection between the outer needle hub 50 and the external passage (not shown) by bending the tube 112.

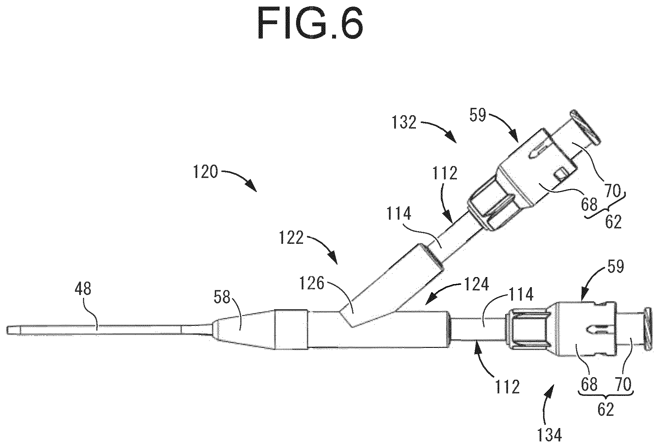

FIG. 6 shows an outer needle assembly 120 as a third practical embodiment of the present invention. An outer needle hub 122 serving as a passage forming member in the present practical embodiment includes a branch member 126 having a branched part 124. In the branched part 124, the main passage extending from the outer needle 48 to the proximal end side is laterally branched, so as to form a branch port part 132 including a branch passage.

Note that FIG. 6 shows a state in which the inner needle (16) is pulled out, the same as in FIGS. 3 and 5. Besides, in the present practical embodiment, the connecting portion between the proximal end side of the outer needle 48 and the outer needle hub 122 (which is near the portion indicated by the reference numeral 58 in FIG. 6) has substantially the same structure as that shown in FIG. 3 in the preceding first practical embodiment. Thus, illustration of the internal structure is omitted. That is, in the present practical embodiment, the internal structure at the connecting portion between the proximal end portion of the outer needle 48 and the outer needle hub 122 is such that the tube 60 in the first practical embodiment is replaced with the branch member 126. More specifically, the distal end portion of the branch member 126 is provided with a large-diameter tube part (corresponding to the large-diameter tube part 84 in FIG. 3) whose inner diameter dimension is enlarged. The proximal end portion (51) of the outer needle 48 is inserted into the said large-diameter tube part of the branch member 126 and is fastened to the branch member 126 (corresponding to the tube 60 in FIG. 3) in an inserted state. Here, the inner diameter dimension of the branch member 126 (corresponding to the inner diameter dimension r1 of the tube 60 in the first practical embodiment) is equal to or smaller than the inner diameter dimension r2 of the proximal end of the outer needle 48, so as to form the small-diameter part (89) at the connecting portion between the proximal end side of the outer needle 48 and the outer needle hub 122. In addition, the connecting portion with the external passage (104) on the proximal end side of the outer needle hub 122 (which is near the portion indicated by the reference numeral 59 in FIG. 6) has substantially the same structure as that shown in FIGS. 3 and 4 in the preceding first practical embodiment. Thus, illustration and description of the internal structure are omitted. Moreover, in the present practical embodiment, since the branch port part 132 provided at the proximal end of the branch passage is substantially the same as a connection port part 134 provided at the proximal end of the main passage, illustration and description of the internal structure are omitted.

Incidentally, on the proximal end side of the branch member 126 (the right side in FIG. 6), the passage connecting member 59 is connected to each of the main passage and the branch passage via the tube 112. The tube 112 has the small-diameter tube part 114 as in the second practical embodiment. Besides, as shown in the enlarged cross-sectional view in FIG. 6, the distal end of the tube 112 is fitted into and fastened to the bore 46 (the main passage) of the branch member 126. In the present practical embodiment, the inner diameter on the distal end side of the tube 112 is enlarged so as to constitute a diameter-enlarged part, and the diameter of the distal end opening of the said diameter-enlarged part is smaller than the inner diameter of the main passage in the branch member 126. With this configuration, as can be seen from the enlarged cross-sectional view in FIG. 6, the connecting portion between the main passage and the tube 112 in the branch member 126 decreases in diameter in an approximately stepwise manner from the distal end side to the proximal end side. Moreover, in the internal passage of the tube 112 as well, the proximal end side of the diameter-enlarged part decreases in diameter in an approximately stepwise manner toward the passage connecting member 59 side.

Indeed, the outer needle assembly 120 of the present practical embodiment also has a small-diameter part (89) located at the connecting portion (88) on the proximal end side of the outer needle 48 and having an approximately stepped shape as in the first practical embodiment. Thus, even if the stepped small-diameter part is not provided on the proximal end side of the branch member 126, the above-mentioned effect of the present invention can be achieved. Therefore, the passage on the proximal end side of the branch member 126 may have an approximately constant inner diameter dimension. Further, since the said passage is remote from the proximal end portion 51 of the outer needle 48, which is enlarged in diameter in a tapered shape, and close to the ventilation path 80, even if the passage is enlarged in diameter from the distal end side toward the proximal end side, the problem of entrainment or remaining of the air in the flowing liquid is unlikely to occur.

While the present invention has been described in detail hereinabove in terms of the practical embodiments, the invention is not limited by the specific description thereof. For example, a valve body and a ventilation path may be provided inside a proximal end connector of an indwelling needle as disclosed in Japanese Unexamined Patent Publication Nos. JP-A-2004-298622 and JP-A-2014-108112, and the configuration of the present disclosure may be adopted.

In the proximal end portion 51 of the outer needle 48, as long as the outer diameter dimension of the second portion 54 connected to the tube 60 is smaller than the outer diameter dimension at the proximal end of the first portion 52, even in the case where the radial width dimension of the stepped surface 86 provided on the inner circumferential surface of the tube 60 is smaller than the thickness dimension of the proximal end of the outer needle 48, diameter enlargement in a stepwise manner at the connecting portion 88 between the outer needle 48 and the tube 60 is avoided.

The outer needle 48 may be connected to the tube 60 so as to be externally fitted thereon. In this case, for example, it is desirable to decrease the outer diameter dimension of the distal end portion of the tube 60 so as to be thin-walled, or to increase the inner diameter dimension of the proximal end portion 51 of the outer needle so as to be thin-walled, or the like, such that the step formed in the connecting portion 88 between the outer needle 48 and the tube 60 is made small. The outer needle 48 may be abutted against the tube 60 in the axial direction and connected by means such as welding.

The tube 60 does not have to have a constant thickness dimension between the needle joining member 58 and the passage connecting member 59, for example. It would also be acceptable that, for example, the inner circumferential surface of the tube 60 gradually protrudes radially inward so as to be thick toward the center in the axial direction.

The outer peripheral surface of the portion of the inner needle 16 to be inserted into the tube 60 can be provided with a slip stopper which makes it difficult to slip on the inner circumferential surface of the tube 60 by, for example, being roughened due to unevenness, grain, etc., or being covered with elastomer, rubber, synthetic resin, etc. having a large frictional resistance. With this configuration, when the tube 60 is gripped, slip between the tube 60 and the inner needle 16 is unlikely to occur, and the relative positions of the inner needle 16 and the outer needle 48 are less likely to be deviated. The configuration that makes it difficult to slip on the inner circumferential surface of the tube 60 (namely, the slip stopper) does not have to be provided on the outer peripheral surface of the inner needle 16, but may be provided on, for example, the inner circumferential surface of the tube 60. That is, the slip stopper is provided between the inner needle 16 and the tube 60, in other words, at least one of the outer peripheral surface of the inner needle 16 and the inner circumferential surface of the tube 60.

The flexible tube is not essential in the passage forming member, and for example, a structure in which the valve body is accommodated in the passage forming member which is made entirely rigid would also be acceptable. As a specific example, it would also be possible to adopt a structure in which the needle joining member and the passage connecting member are directly fixed to each other without interposing the tube, or the like.

As shown in the preceding practical embodiment, it is desirable that the ventilation path 80 is provided with the ventilation filter 82 for preventing blood leakage. However, for example, in the case where blood leakage is prevented by the cross-sectional area, the cross-sectional shape, and the like of the ventilation path, the ventilation filter may be omitted. Besides, the ventilation path is not limited to the structure extending between the components of the passage forming member, and may have, for example, a hole structure penetrating the components of the passage forming member.

### KEYS TO SYMBOLS

10 outer needle assembly (first practical embodiment)
12 indwelling needle assembly
14 inner needle assembly
16 inner needle
18 blade surface
20 needle tip
22 inner needle hub
24 pedestal part
26 protector accommodation part
28 linking part
30 restricting tube part
32 inner needle cap
33 film-like filter
34 protector housing
36 accommodation tube part
38 lid body
40 needle tip protector
42 shielding member
44 fixing member
46 bore (internal space)
48 outer needle
50 outer needle hub (passage forming member)
51 proximal end portion
52 first portion
54 second portion
55 distal end portion
56 puncture part
57 tapered part
58 needle joining member
59 passage connecting member
60 tube
62 valve housing
64 valve body
66 pusher
68 cover member
70 pusher guide
72 tube linking member
73 tube fixing part
73a tapered surface
73b proximal end tube part
74 male screw part
76 slit
77 valve support member
78 first internal space
79 second internal space
80 ventilation path
82 ventilation filter
84 large-diameter tube part
86 stepped surface
88 connecting portion
89 small-diameter part
90 connector cap
92 bottom wall
94 pipe-shaped projection
96 peripheral wall
98 elastic piece
100 female thread
102 locking projection
104 external passage
106 male connector
110 outer needle assembly (second practical embodiment)
112 tube
114 small-diameter tube part
116 thick-walled linking part
120 outer needle assembly (third practical embodiment)
122 outer needle hub (passage forming member)
124 branched part
126 branch member
132 branch port part
134 connection port part

## Claims

1. An outer needle assembly (10, 110, 120) including:
an outer needle (48) of hollow shape through which an inner needle (16) is insertable;
a passage forming member (50, 122) connected to a proximal end side of the outer needle (48) in a communicating state, wherein a bore (46) is penetrated in an axial direction through the outer needle (48) and the passage forming member (50, 122);
a valve body (64) arranged inside the passage forming member (50, 122); and
a ventilation path (80) provided to the passage forming member (50, 122) and placing a first internal space (78) in the bore (46) on a distal end side with respect to the valve body (64) in communication with an outside;
a proximal end portion (51) of the outer needle (48) having a tapered shape that is enlarged in diameter toward a proximal end,
**characterized in that** in a connecting portion (88) between the outer needle (48) and the passage forming member (50, 122), an inner diameter dimension of the passage forming member (50, 122) is equal to or smaller than that of the outer needle (48).

2. The outer needle assembly (10, 110, 120) according to claim 1, wherein in the connecting portion (88) between the outer needle (48) and the passage forming member (50, 122), the inner diameter dimension of the passage forming member (50, 122) is equal to that of the outer needle (48).

3. The outer needle assembly (10, 110, 120) according to claim 1 or 2, wherein the passage forming member (50, 122) includes a flexible tube (60, 112), and the flexible tube (60, 112) is connected to the outer needle (48).

4. The outer needle assembly (10, 110) according to claim 3, wherein a distal end portion of the flexible tube (60, 112) includes a large-diameter tube part (84) whose inner diameter dimension is increased, and a proximal end portion (51) of the outer needle (48) is fastened to the large-diameter tube part (84) in an inserted state.

5. The outer needle assembly (110) according to claim 4, wherein the flexible tube (112) includes a small-diameter tube part (114) provided on the proximal end side with respect to the large-diameter tube part (84), the small-diameter tube part (114) having an inner diameter dimension and an outer diameter dimension that are smaller than those of the large-diameter tube part (84).

6. The outer needle assembly (10, 110, 120) according to any one of claims 1,4 and 5, wherein in the connecting portion (88) between the outer needle (48) and the passage forming member (50, 122), the inner diameter dimension of the passage forming member (50, 122) is equal to that of the outer needle (48), and the passage forming member (50, 122) includes a flexible tube (60, 112), and an inner circumferential surface constituting the bore (46) ranging from a distal end of the outer needle (48) to a proximal end of the flexible tube (60, 112) is not provided with a stepped diameter-enlarged portion.

7. The outer needle assembly (10, 110, 120) according to any one of claims 1-6, wherein:
the first internal space (78) in the bore (46) is longer than a second internal space (79) on a proximal end side with respect to the valve body (64), and
the first internal space (78) comprises:
a puncture part (56) formed in a portion of the outer needle (48), the portion being configured for puncturing a blood vessel;
a tapered part (57) formed on the proximal end side with respect to the puncture part (56), the tapered part (57) having an inner diameter dimension which increases toward the proximal end side; and
a small-diameter part (89) formed on the proximal end side with respect to the tapered part (57), the small-diameter part (89) having an inner diameter dimension which is smaller than or equal to that of a proximal end of the tapered part (57).

## Patentansprüche

1. Außennadelanordnung (10, 110, 120), enthaltend:
eine Außennadel (48) mit Hohlform, durch die eine Innennadel (16) einführbar ist;
ein kanalbildendes Element (50, 122), das in einem kommunizierenden Zustand mit einer proximalen Endseite der Außennadel (48) verbunden ist, wobei eine Bohrung (46) in einer axialen Richtung durch die Außennadel (48) und das kanalbildende Element (50, 122) durchgestochen ist;
einen Ventilkörper (64), der im Inneren des kanalbildenden Elements (50, 122) angeordnet ist; und
einen Lüftungsweg (80), der an dem kanalbildenden Element (50, 122) bereitgestellt ist und eine Kommunikation von einem ersten Innenraum (78) in der Bohrung (46) auf einer distalen Endseite in Bezug auf den Ventilkörper (64) mit einer Außenseite herstellt;
einen proximalen Endabschnitt (51) der Außennadel (48) mit einer konischen Form, dessen Durchmesser zu einem proximalen Ende hin vergrößert ist,
**dadurch gekennzeichnet, dass**
in einem Verbindungsabschnitt (88) zwischen der Außennadel (48) und dem kanalbildenden Element (50, 122) eine Innendurchmesserabmessung des kanalbildenden Elements (50, 122) gleich oder kleiner als diejenige der Außennadel (48) ist.

2. Außennadelanordnung (10, 110, 120) nach Anspruch 1, wobei in dem Verbindungsabschnitt (88) zwischen der Außennadel (48) und dem kanalbildenden Element (50, 122) die Innendurchmesserabmessung des kanalbildenden Elements (50, 122) gleich derjenigen der Außennadel (48) ist.

3. Außennadelanordnung (10, 110, 120) nach Anspruch 1 oder 2, wobei das kanalbildende Element (50, 122) ein flexibles Rohr (60, 112) enthält und das flexible Rohr (60, 112) mit der Außennadel (48) verbunden ist.

4. Außennadelanordnung (10, 110) nach Anspruch 3, wobei ein distaler Endabschnitt des flexiblen Rohrs (60, 112) einen Rohrteil (84) mit großem Durchmesser enthält, dessen Innendurchmesserabmessung erhöht ist, und ein proximaler Endabschnitt (51) der Außennadel (48) in einem eingeführten Zustand an dem Rohrteil (84) mit großem Durchmesser befestigt ist.

5. Außennadelanordnung (110) nach Anspruch 4, wobei das flexible Rohr (112) einen Rohrteil (114) mit kleinem Durchmesser enthält, der an der proximalen Endseite in Bezug auf den Rohrteil (84) mit großem Durchmesser bereitgestellt ist, wobei der Rohrteil (114) mit kleinem Durchmesser eine Innendurchmesserabmessung und eine Außendurchmesserabmessung aufweist, die kleiner als diejenigen des Rohrteils (84) mit großem Durchmesser sind.

6. Außennadelanordnung (10, 110, 120) nach einem der Ansprüche 1, 4 und 5,
wobei in dem Verbindungsabschnitt (88) zwischen der Außennadel (48) und dem kanalbildenden Element (50, 122) die Innendurchmesserabmessung des kanalbildenden Elements (50, 122) gleich derjenigen der Außennadel (48) ist und das kanalbildende Element (50, 122) ein flexibles Rohr (60, 112) enthält und eine die Bohrung (46) darstellende innere Umfangsoberfläche, die von einem distalen Ende der Außennadel (48) zu einem proximalen Ende des flexiblen Rohrs (60, 112) reicht, nicht mit einem abgestuften, durchmesservergrößerten Abschnitt versehen ist.

7. Außennadelanordnung (10, 110, 120) nach einem der Ansprüche 1-6, wobei:
der erste Innenraum (78) in der Bohrung (46) länger als ein zweiter Innenraum (79) an einer proximalen Endseite in Bezug auf den Ventilkörper (64) ist und
der erste Innenraum (78) Folgendes umfasst:
einen Punktionsteil (56), der in einem Abschnitt der Außennadel (48) ausgebildet ist, wobei der Abschnitt zum Punktieren eines Blutgefäßes konfiguriert ist;
einen konischen Teil (57), der an der proximalen Endseite in Bezug auf den Punktionsteil (56) ausgebildet ist, wobei der konische Teil (57) eine Innendurchmesserabmessung aufweist, die zu der proximalen Endseite hin zunimmt; und
einen Teil (89) mit kleinem Durchmesser, der an der proximalen Endseite in Bezug auf den konischen Teil (57) ausgebildet ist, wobei der Teil (89) mit kleinem Durchmesser eine Innendurchmesserabmessung aufweist, die kleiner oder gleich derjenigen eines proximalen Endes des konischen Teils (57) ist.

## Revendications

1. Ensemble aiguille externe (10, 110, 120) comprenant :
une aiguille externe (48) de forme creuse à travers laquelle une aiguille intérieure (16) peut être insérée ;
un élément formant passage (50, 122) relié à un côté d'extrémité proximale de l'aiguille externe (48) dans un état de communication, dans lequel un alésage (46) est pénétré dans une direction axiale à travers l'aiguille externe (48) et l'élément formant passage (50, 122) ;
un corps de soupape (64) agencé à l'intérieur de l'élément formant passage (50, 122) ; et
un chemin de ventilation (80) ménagé pour l'élément formant passage (50, 122) et plaçant un premier espace interne (78) dans l'alésage (46) d'un côté d'extrémité distale par rapport au corps de soupape (64) en communication avec un extérieur ;
une partie d'extrémité proximale (51) de l'aiguille externe (48) présentant une forme conique dont le diamètre est agrandi vers une extrémité proximale, **caractérisé en ce que**
dans une partie de liaison (88) entre l'aiguille externe (48) et l'élément formant passage (50, 122), une dimension de diamètre intérieur de l'élément formant passage (50, 122) est égale ou inférieure à celle de l'aiguille externe (48).

2. Ensemble aiguille externe (10, 110, 120) selon la revendication 1, dans lequel dans la partie de liaison (88) entre l'aiguille externe (48) et l'élément formant passage (50, 122), la dimension du diamètre intérieur de l'élément formant passage (50, 122) est égale à celle de l'aiguille externe (48).

3. Ensemble aiguille externe (10, 110, 120) selon la revendication 1 ou 2, dans lequel l'élément formant passage (50, 122) comprend un tube souple (60, 112), et le tube souple (60, 112) est relié à l'aiguille externe (48).

4. Ensemble aiguille externe (10, 110) selon la revendication 3, dans lequel une partie d'extrémité distale du tube souple (60, 112) comprend une pièce de tube de grand diamètre (84) dont la dimension de diamètre intérieur est augmentée, et une partie d'extrémité proximale (51) de l'aiguille externe (48) est fixée à la pièce de tube de grand diamètre (84) dans un état inséré.

5. Ensemble aiguille externe (110) selon la revendication 4, dans lequel le tube souple (112) comprend une pièce de tube de petit diamètre (114) ménagée du côté d'extrémité proximale par rapport à la pièce de tube de grand diamètre (84), la pièce de tube de petit diamètre (114) présentant une dimension de diamètre intérieur et une dimension de diamètre externe qui sont plus petites que celles de la pièce de tube de grand diamètre (84).

6. Ensemble aiguille externe (10, 110, 120) selon l'une quelconque des revendications 1, 4 et 5,
dans lequel, dans la partie de liaison (88) entre l'aiguille externe (48) et l'élément formant passage (50, 122), la dimension du diamètre intérieur de l'élément formant passage (50, 122) est égale à celle de l'aiguille externe (48), et l'élément formant passage (50, 122) comprend un tube souple (60, 112), et une surface circonférentielle intérieur constituant l'alésage (46) allant d'une extrémité distale de l'aiguille externe (48) à une extrémité proximale du tube souple (60, 112) n'est pas dotée d'une partie élargie à diamètre étagé.

7. Ensemble aiguille externe (10, 110, 120) selon l'une quelconque des revendications 1 à 6, dans lequel :
le premier espace interne (78) dans l'alésage (46) est plus long qu'un second espace interne (79) d'un côté d'extrémité proximale par rapport au corps de soupape (64), et
le premier espace interne (78) comprend :
une pièce de percée (56) formée dans une partie de l'aiguille externe (48), la partie étant conçue pour percer un vaisseau sanguin ;
une pièce conique (57) formée du côté d'extrémité proximale par rapport à la pièce de percée (56), la pièce conique (57) présentant une dimension de diamètre intérieur qui augmente vers le côté d'extrémité proximale ; et
une pièce de petit diamètre (89) formée du côté d'extrémité proximale par rapport à la pièce conique (57), la pièce de petit diamètre (89) présentant une dimension de diamètre intérieur qui est inférieure ou égale à celle d'une extrémité proximale de la pièce conique (57).
